# EUROPEAN PATENT SPECIFICATION

(11) **EP 3 197 964 B1**
(45) Date of publication and mention of the grant of the patent: **15.01.2020**
(21) Application number: 15813587.1
(22) Date of filing: 28.09.2015
(51) Int. Cl.: C09D 163/00, A61L 27/00

(54) **MEDICAL COMPOSITION**
MEDIZINISCHE BESCHICHTUNGSZUSAMMENSETZUNG
COMPOSITION DE REVÊTEMENT MÉDICAL

(30) Priority: 26.09.2014 EP 14186637
(43) Date of publication of application: 02.08.2017
(73) Proprietor: DSM IP Assets B.V., 6411 TE Heerlen (NL); Universiteit Maastricht, 6211 LK Maastricht (NL); Academisch Ziekenhuis Maastricht, 6229 HX Maastricht (NL)
(72) Inventor: DIRKS, Antonius Johannes, NL-6100 AA Echt (NL); LOONTJENS, Jacobus Antonius, NL-6100 AA Echt (NL); RIJK, Llewellyn, NL-6100 AA Echt (NL); ODEKERKEN, Jim C.E., NL-6100 AA Echt (NL); WELTING, Tim Johannes Maria, NL-6100 AA Echt (NL)
(74) Representative: DSM Intellectual Property
(86) International application number: PCT/NL2015/050671
(87) International publication number: WO 2016/048155

(56) References cited:
- US-A1- 2014 010 858

## Description

### Field

The invention is directed to a composition, a coating prepared from said composition, an implant comprising a substrate comprising said coating, a method for preparing an implant with said coating, and a method for improving the bone apposition onto an implant.

### Background

The use of prosthetic implants e.g. for replacing or supplementing fractured, damaged, or degenerated skeletal bone in a mammalian body is commonplace in the medical arts. Usually, the prosthetic implant device is made of a biocompatible metal such as stainless steel, cobalt-chromium-molybdenum alloy, tungsten, titanium, aluminium, cobalt-chromium-tungsten-nickel, and similar alloys. However, also various synthetic plastics are being investigated and applied. Most often, the prosthetic implant device is intended to become a permanent part of the skeletal structure.

Several problems are prevalent when implanting items in the human body. These problems include infection, formation of biofilms, and improper integration of the implant into the bone tissue. The problem of improper integration is especially prevalent where the prosthesis is subject to large functional loads and shear stresses. This difficulty in achieving a prosthesis that is strongly bonded to the bone, and which can withstand large shear and tensile stress loads, has led to the development of a variety of attachment mechanisms. Many of these mechanisms attempt to adaptively reform bone around the prosthesis, with the newly formed bone eventually bonding to the outer surface of the implant.

It is common to coat implants for protection of the underlying implant material, to improve the properties of the implant at the outer surface, or to impart a desired effect, such as anti-microbial, antiseptic or improved tissue integration effect. Many such coatings that are presently commercially available are inorganic. A common way to provide some bone growth on an implant and thereby improve its attachment is to coat the implant with hydroxyapatite. Although such inorganic implant coatings may have certain advantages, they may be brittle, difficult to apply, or offer insufficient anti-microbial, anti-septic, or tissue integration effects.

WO-A-99/030672 discloses a multi-layer implant coating comprising organic components having purported good growth of osteogenic cells. The organic layers may be epoxy resins, epoxy resins modified with "acetethylamin" (original German text), epoxy resins modified with chromic acid (or chromo-sulphuric acid), glutardialdehyde, polyvinylacetate, polyacrylonitriles, polyhydroxybutyrates, polyacrylates, polyoxymethylenes, polystyrenes, polymethylenes, polyethylenes, paraffin, polymethyl methacrylates, acetylcelluloses, nitrocelluloses, and polyvinylpyrrolidone. The outer layers may be the above organic compounds, hydroxyapatite, proteins, in particular albumin, trypsin, pepsin, and collages. The outer layer is treated with a tissue culture plasma treatment.

Geckeler et al. (Cellular Physiology and Biochemistry 2003, 13(3), 155-164) report that excellent biocompatibility to SAOS-2 osteoblastic cells can be obtained with hydrophobic surfaces generated for instance by epoxy resins. Chemical modification of epoxy resin surfaces were reported as yielding even a further increased viability index surpassing the viability index obtained with cell culture vessels.

US 2014/0010858 A1 discloses polymeric coating compositions comprising a biodegradable copolymer and implantable devices formed thereof.

Despite these efforts known from the prior art, there remains a need for further and alternative approaches in improving the coating of implants.

### Summary

The inventors surprisingly found that certain problems of the prior art can be overcome by a specifically designed composition that can be used for coating an implant. This composition may form a coating on an implant that contains organic substituents, is capable of easy application to an implant surface, and may provide an improved anti-microbial, anti-septic, or tissue integration effect.

Accordingly, in a first aspect the invention is directed to a composition comprising:
(A) a compound comprising two or more epoxy groups;
(B) an aliphatic amine comprising one or more selected from a primary amine group and a secondary amine group; and
(C) one or more compounds selected from general formula (1), (2), (3), and (4) 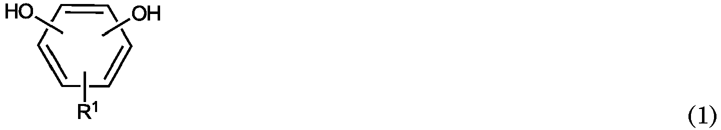 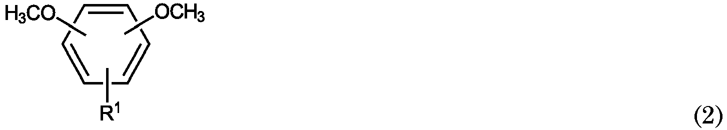 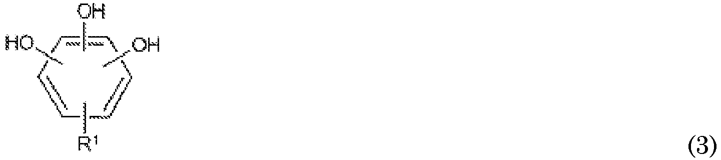 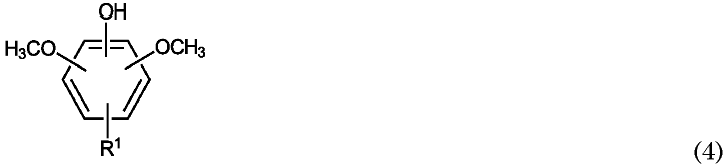
   wherein R¹ is a group comprising a primary amine group, a secondary amine group, a carboxyl group, and/or a thiol group, and
   wherein the sum of the number of epoxy groups in component (A) and the number of primary amine groups in component (B) is equal to three or greater.

In a further aspect the invention is directed to a coating composition comprising a composition of the invention.

In a further aspect the invention is directed to a coating prepared from a composition of the invention.

In yet a further aspect the invention is directed to a medical device, such as an implant, comprising a substrate comprising a coating of the invention.

In yet a further aspect the invention is directed to a method for preparing an implant with a coating, the method comprising:
- coating the implant with a composition according to the invention,
- curing the composition, thereby forming a coating, and
- optionally, post-curing the coating.

In yet a further aspect the invention is directed to a method for improving the bone apposition onto an implant, comprising coating said implant with a composition according to the invention.

In yet a further aspect, the invention is directed to a bone cement comprising the composition according to the invention.

In yet a further aspect, the invention is directed to an article formed from the composition according to the invention.

In yet a further aspect, the invention is directed to a liquid composition for three-dimensional printing, a method of forming a three-dimensional object, and a three-dimensional object formed via three-dimensional printing.

In yet a further aspect, the invention is directed to a coating or an article formed from components (A) and (B), wherein the sum of the number of epoxy groups in component (A) and the number of primary amine groups in component (B) is equal to three or greater, and such that the formed coating or article has epoxy groups on its surface. The coating or article is treated with component (C) after forming the article or coating, thereby reacting the primary amine group, secondary amine group, carboxyl group, or thiol group of component (C) with the free epoxy group on the surface of the coating or article.

The composition according to the invention may allow for a coated implant with increased percentage of bone apposition. An increased percentage of bone apposition improves the integration of the implant *in vivo* and results in an enhanced attachment between implant and bone. Hence, the composition of the invention may achieve tissue-integration of implants. Up to now such integration was only possible by selectively choosing the implant (surface) material (*e.g.* grind-blasted titanium) or by the application of inorganic calcium-phosphate coatings (*e.g.* hydroxyapatite). Polydopamine coatings have been mentioned as potentially further increasing the osteointegration of polydimethylsiloxane-polycaprolactone bone regeneration scaffolds (Jimenez-Vergara et al., Society for Biomaterials 2013, "Polydopamine-coated PDMS-PCL shape memory polymer foams for bone regeneration", abstract #239.

These prior solutions may not be suitable for certain types of polymeric implant materials, may be brittle or lack other useful mechanical properties, and may require the use of an application method that is undesirable. The composition of the invention may be applied to a large number of implant substrates, including polymeric implant materials, and ceramic and metallic surfaces.

### Detailed Description

All amounts of components stated as being present in a % by weight of the total composition are based on the total weight of the composition excluding any solvent (F). All amounts of components stated as being present in a % by weight of the composition including solvent is based on the weight of the composition including solvent (F).

The composition of the invention comprises a compound comprising two or more epoxy groups (A). The terms "a compound comprising two or more epoxy groups" or "the epoxy" as used in this application are meant to refer to any material, monomeric, oligomeric, polymeric or resinous, which contains two or more oxirane groups.

The epoxy may be saturated or unsaturated, aliphatic, cycloaliphatic, heterocyclic or aromatic, and may be substituted, if desired, with substituents such as halogens, sulfur, ester groups, urethane groups, hydroxy groups, mercapto groups, amino groups, ether groups, acid or acid anhydride groups, ketone or aldehyde groups, or the like. It is preferred that the epoxy is an aliphatic or cycloaliphatic epoxy. The epoxy resin preferably does not contain a bisphenol A moiety.

Preferably the epoxy (A) is selected from the group consisting of 1,4-butanediol diglycidyl ether, 1,4-cyclohexanedimethanol diglycidyl ether, 4-vinylcyclohexene diepoxide, ethyleneglycol diglycidyl ether, hydrogenated diglycidyl ether of bisphenol A, glycerol diglycidyl ether, neopentylglycol diglycidyl ether, poly(ethyleneglycol) diglycidyl ether, and poly(propyleneglycol) diglycidyl ether. More preferably, the epoxy is selected from the group consisting of 1,4-cyclohexanedimethanol diglycidyl ether, hydrogenated diglycidyl ether of bisphenol A, and glycerol diglycidyl ether.

The compound (A) may also comprise any combination of the exemplified compounds mentioned above.

Typically, the amount of epoxy resin (A) can be 10-90 % by weight of the total composition, such as 10-80 % by weight of the total composition, 10-70% by weight of the total composition, 10-60 % by weight of the total composition, 10-50 % by weight of the total composition, 15-50 % by weight of the total composition, or 15-40 % by weight of the total composition.

The composition of the invention further comprises an amine (B) comprising one or more selected from a primary amine group and a secondary amine group. The term "comprising one or more selected from a primary amine group and a secondary amine group" or "the amine" as used in this application is meant to refer to an organic compound that comprises either one or more primary amine groups, or one or more secondary amine groups, or a combination of one or more primary amine groups and one or more secondary amine groups. Suitable examples include compounds that can be represented by general formula H₂N-R-NH₂, wherein R can be selected from optionally substituted linear or cyclic alkylene.

The amine (B) is an aliphatic amine. Apart from the primary and/or secondary amine nitrogen atom(s), the amine may further contain one or more heteroatoms selected from nitrogen and oxygen. The amine (B) can, for instance, further comprise a tertiary amine in the molecule. In an embodiment, the amine does not contain any heteroatoms other than the primary and/or secondary amine nitrogen atoms.

The amine can comprise one or more primary amine groups. In an embodiment, the amine comprises two or more primary amine groups. In an embodiment, the amine comprises two primary amine groups. In an embodiment, the amine comprises one primary amine group and one secondary amine group. In an embodiment, the amine comprises three primary amine groups.

Preferably, the amine is selected from the group consisting of 1,4-*bis*(3-aminopropyl)piperazine, 3,3'-diamino-*N*-methyl dipropylamine, 4,7,10-trioxa-1,13-tridecanediamine, ethylenediamine, isophorone diamine, spermidine, and *N*-(2-aminoethyl)-1,3-propanediamine. Even more preferably, the amine is isophorone diamine.

In an embodiment, the amine is an amine functionalised amino acid, such as a diamine functionalised amino acid.

The amine (B) may also comprise any combination of the exemplified compounds mentioned above.

The amine may have a molecular weight in the range of 50-300 g/mol, such as in the range of 50-250 g/mol.

The amount of the amine (B) in the composition of the invention can be 3-50 % by weight of the total composition, 3-40 % by weight of the total composition, 3-30 % by weight of the total composition, such as 3-20 % by weight of the total composition, or 4-15 % by weight of the total composition.

In accordance with a composition according to the invention, the sum of the number of epoxy groups in component (A) and the number of primary amine groups in component (B) is equal to three or greater. Suitably, the sum of the number of epoxy groups in component (A) and the number of primary amine groups in component (B) is equal to four or greater. In an embodiment, the sum of the number of epoxy groups in component (A) and the number of primary amine groups in component (B) is 3 or more, such as 4 or more. In an embodiment, the sum of the number of epoxy groups in component (A) and the number of primary amine groups in component (B) 10 or less, such as 6 or less, or 5 or less. Hence, the sum of the number of epoxy groups in component (A) and the number of primary amine groups in component (B) can, for instance, be in the range of 4-10, in the range of 3-6, in the range of 4-6, in the range of 3-5, or in the range of 4-5.

Component (C) is one or more compounds selected from general formula (1), (2), (3), or (4). 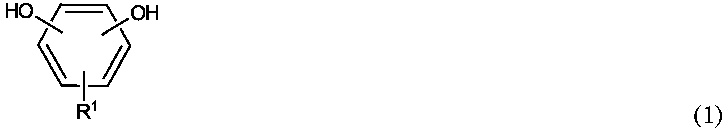 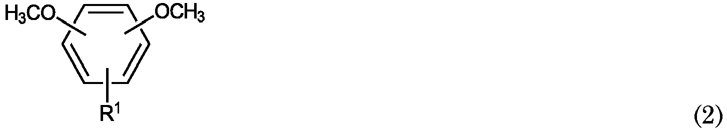 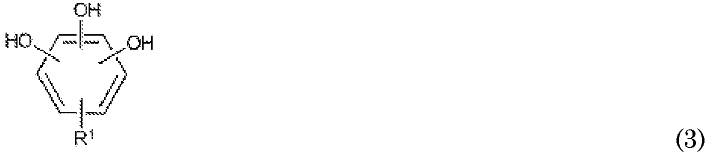 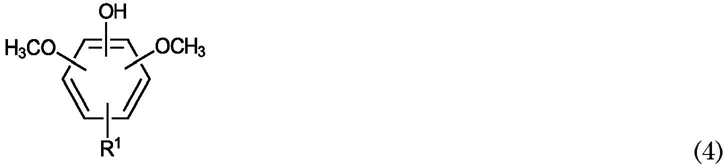 wherein R¹ is a group comprising a primary amine group, a secondary amine group, a carboxyl group, and/or a thiol group. R¹ can be a group where a carbon atom is attached to the aromatic ring of general formula (1), (2), (3), or (4) and comprising a primary group, a secondary amine group, a carboxyl group, and/or a thiol group. Preferably, component (C) is a compound selected from general formula (1) or (3). Preferably, component (C) is a compound selected from general formula (1) or (3) and R¹ comprises a group comprising a primary and/or secondary amine group. Preferably, component (C) is a compound selected from general formula (1) or (3) and R¹ comprises a group where a carbon atom is attached to the aromatic ring of general formula (1) or (3) and comprising a primary and/or secondary amine group. Preferably, component (C) is a compound of general formula (1). Preferably, component (C) is a compound of general formula (1) and R¹ comprises a group comprising a primary and/or secondary amine group. Preferably, component (C) is a compound of general formula (1) and R¹ comprises a group where a carbon atom is attached to the aromatic ring of general formula (1) and comprising a primary and/or secondary amine group.

In certain preferred embodiments, the compound (C) is selected from a catechol derivative, a guaiacol derivative or a syringol derivative. In certain preferred embodiments, the compound (C) is selected from a catechol derivative. In an embodiment, the compound (C) is a compound according to general formula (5) 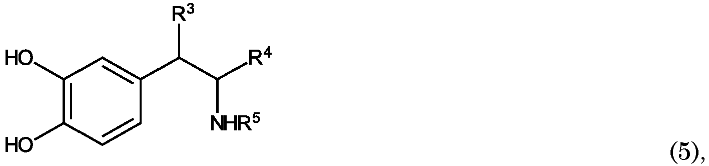 wherein
R3 is H or OH,
R⁴ is H or COOH, and
R⁵ is H or CH₃.

In an embodiment wherein the compound (C) is according to formula (3), each of R³, R⁴ and R⁵ are H. In a further embodiment wherein the compound (C) is according to formula (3), R³ is OH and R⁴ and R⁵ are both H. In yet a further embodiment wherein the compound (C) is according to formula (3), R³ is OH, R⁴ is H, and R⁵ is CH₃. In yet a further embodiment wherein the compound (C) is according to formula (3), R³ and R⁵ are H, and R⁴ is COOH.

Some examples of catechol amines according to general formula (1) and (5) are provided below.

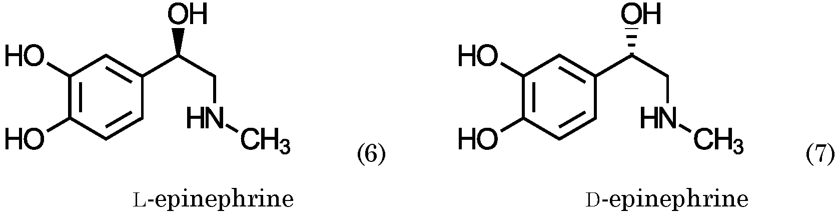

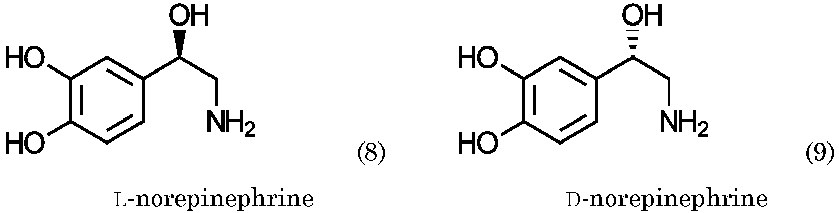

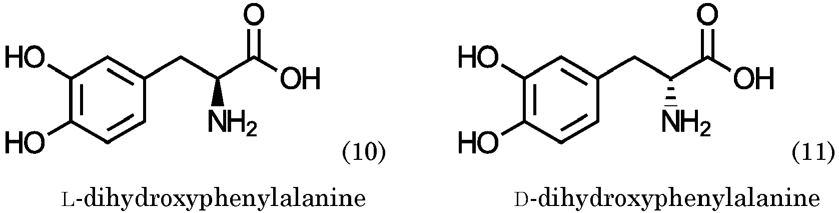

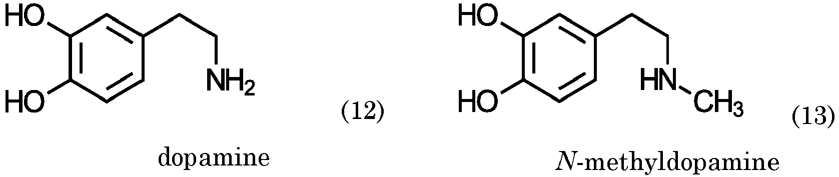

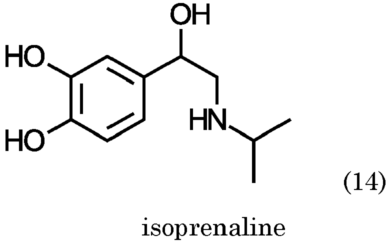

Preferred examples of catechol amines according to general formula (1) and/or (5) include epinephrine, norepinephrine, N-methyldopamine, dopamine, and L-dihydroxyphenylalanine (L-DOPA). A preferred catechol amine according to general formula (3) is dopamine.

In an embodiment, the compound (C) is 2-(3',4'-dihydroxy-phenyl)-morpholine, which is shown below as general formula (15).

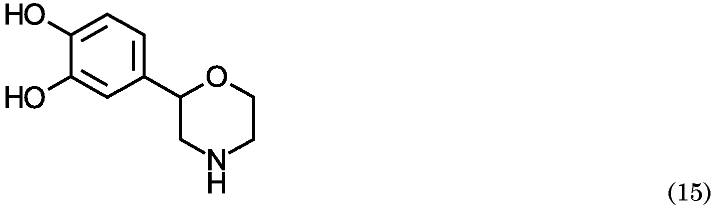

In an embodiment, the compound (C) is a compound according to general formula (3). In an embodiment, the compound (C) is 5-(2-aminoethyl)-1,2,3-benzenetriol, which is shown below as general formula (16).

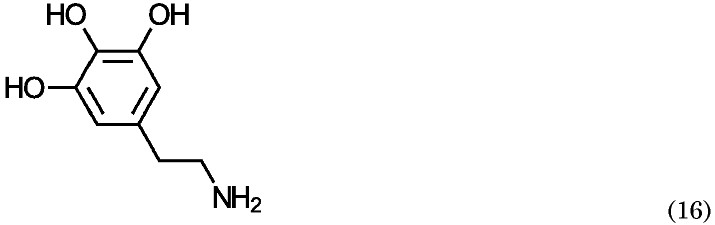

The compound (C) may also comprise any combination of the exemplified compounds mentioned above.

The compound (C) may be added or present in the composition in the form of an appropriate salt. An example of a suitable salt is dopamine hydrochloride.

The amount of the compound (C) in the composition of the invention can be 1-25 % by weight of the total composition, such as 1-20 % by weight of the total composition, 1-15 % by weight of the total composition, 5-25 % by weight of the total composition, 5-20 % by weight of the total composition, 5-15 % by weight of the total composition or 2-10 % by weight of the total composition.

In an embodiment, the composition of the invention is prepared by first dissolving the amine (B) in a solvent, such as ethanol. Next, the component (C) is added and fully dissolved. Lastly, the epoxy (A) is added.

In an embodiment, the composition of the invention is prepared by first dissolving the amine (B) in a first solvent, such as ethanol. Next, the component (C) is added and fully dissolved. The epoxy (A) is dissolved in a second solvent, which may be the same as or different than the first solvent, and is miscible in the first solvent. In an embodiment, the two solutions are mixed either as a liquid or aerosol and then applied to the substrate. In an embodiment, these two solutions are jetted in a three-dimensional printing process.

In the case that R¹ is a group comprising a carboxyl group, the amount of carboxyl groups in the composition is preferably 10 mol% or less of the total amount of primary and secondary amine groups in the composition.

For purposes of calculating the equivalent ratio of all primary and secondary amine groups to all epoxy groups in the instant application, a primary amine group is counted twice, while a secondary amine group and an epoxy group are counted once. For example, a composition with 10 primary amine groups, 5 secondary amine groups, and 10 epoxy groups would have an equivalent ratio of (20 + 5) / 10 = 2.5. In an embodiment, the equivalent ratio of all primary and secondary amine groups to all epoxy groups (*e.g.* all primary and secondary amine groups divided by all epoxy groups) in the total composition is in the range of 0.95-1.10, preferably in the range of 1.00-1.08. In an embodiment, the equivalent ratio of all primary amine groups to all epoxy groups in the composition is in the range of 0.95-1.10, preferably in the range of 1.00-1.08.

Apart from the essential components (A), (B), and (C), the composition of the invention can further comprise additional optional components.

For example, the composition of the invention can further comprise an antimicrobial agent (D). An antimicrobial agent is an antibiotic, antimicrobial, antiseptic and/or antifungal compound. Preferably, the antimicrobial agent (D) is an antimicrobial compound and/or an antiseptic compound. The antimicrobial agent may provide the composition of the invention, or a coating or article formed from the composition, with an antimicrobial functionality. Advantageously, the composition of the invention is such as to readily allow the use of a wide range of antimicrobial agents.

The compositions of the invention may be formulated such as to allow a local delivery of the antimicrobial agent (D), or of other drugs.

Many different antimicrobial agents can suitably be used in the composition of the invention. The antimicrobial agent can comprise one or more of a tetracycline, a biguanide (including bisbiguanides), elemental silver such as silver nanoparticles, silver nitrate, silver oxide, silver salts, silver sulfadiazine, silver zeolites, triclosan, antifolates, aminoglycosides, carbapenems, cephalosporins, fluoroquinolines, glycopeptides, tuberculostatics, macrolides, monobactams, oxazolidinones, penicillin, sulphonamide, and/or their salts. Preferred antimicrobial agents are silver nanoparticles, metallic silver, and/or ionic silver.

The antimicrobial agent can comprise one or more of chlorhexidine, alexidine, methylisothiazolone (2-methylisothiazolone hydrochloride), thymol (5-methyl-2 isopropyl phenol), α-terpineol (α-α-4-trimethyl-3-cyclohexine-1-methanol), cetylpyridinium chloride (1-hexadecylpyridinium chloride), and chloroxylenol (4-chloro, 3,5-dimethyl phenol).

Preferably, the composition of the invention comprises chlorhexidine. Included in the definition of chlorhexidine are pharmaceutically acceptable salts of chlorhexidine. Chlorhexidine, such as chlorhexidine gluconate or chlorhexidine acetate, is a biguanide with a very rapid bactericidal activity against a broad range of microorganisms, including gram-positive bacteria (such as *Staphylococci, Enterococcus* species), gram-negative bacteria (such as *Escherichia coli* and *Pseudomonas aeruginosa*) and *Candida* species. Chlorhexidine causes disruption of microbial cell membranes and precipitation of cellular contents, and its effectiveness is not affected by the presence of organic matter, such as blood. An important attribute of chlorhexidine is its prolonged persistence on the skin, which is beneficial for reducing infections related to medical devices that are usually caused by organisms migrating from skin, such as vascular catheter and orthopaedic device-related infections. Chlorhexidine is generally not stable for a significant amount of time in a composition at a pH of 8 or more. Chlorhexidine has been used extensively as a skin cleanser for over 20 years, and also has been used to coat vascular catheters.

The antimicrobial agent can be used individually or in combinations of two or more of them to obtain a synergistic effect. Some examples of combinations of antimicrobial agents include a mixture of chlorhexidine, methylisothiazolone and α-terpineol; thymol and chloroxylenol; thymol and methylisothiazolone; chlorhexidine and cetylpyridinium chloride; chlorhexidine and chloroxylenol; or chlorhexidine, methylisothiazolone and thymol. These combinations provide a broad spectrum of activity against a wide variety of organisms. However, other combinations of antimicrobial agents (D) may be applied as well.

An antimicrobial agent can also be added to an article or coating after forming the article or coating from a composition. For example, a coating or article formed from a composition of the invention may be dipped in a mixture comprising silver, such as silver nanoparticles, metallic silver, and/or ionic silver, and a solvent.

The amount of the antimicrobial agent (D) in the composition of the invention can be 0-15 % by weight of the total composition, such as 0-8 % by weight of the total composition or 0-5 % by weight of the total composition. Suitably, the amount of antimicrobial agent (D) in the composition of the invention can be 0.05-10 % by weight of the total composition, such as 0.05-8 % by weight of the total composition, 0.1-5 % by weight of the total composition. Typically, the amount of each antimicrobial agent used is sufficient to form an effective concentration to inhibit the growth of bacterial and fungal organisms, such as *Staphylococci*, gram-positive bacteria, gram-negative bacteria and *Candida.*

A further optional component in the composition of the invention is a bone growth promoter (E). The bone growth promoter can comprise an osteoconductive and/or osteoinductive agent.

Suitable examples of osteoinductive agents include bone morphogenetic proteins (BMP, such as BMP 1, BMP 3, BMP 4, and BMP 7); demineralised bone matrix, various growth factors known to be osteoinductive (*e*.*g*. transforming growth factor-α, growth and differentiation growth factor), stem cells or those with osteoblastic potential, *etc.* For example, growth factors can be selected from the group consisting of platelet-derived growth factor (PDGF), platelet-derived angiogenesis factor (PDAF), vascular endothelial growth factor (VEGF), platelet-derived epidermal growth factor (PDEGF), platelet factor 4 (PF-4), transforming growth factor β (TGF-β), acidic fibroblast growth factor (FGF-α), basic fibroblast growth factor (FGF-β), transforming growth factor (TGF-α), insulin-like growth factors 1 and 2 (IGF-1 and IGF-2), B thromboglobulin-related proteins (BTG), thrombospondin (TSP), fibronectin, von Willebrand factor (vWF), fibropeptide A, fibrinogen, albumin, plasminogen activator inhibitor 1 (PAI-1), osteonectin, regulated upon activation normal T cell expressed and presumably secreted (RANTES), gro-A, vitronectin, fibrin D-dimer, factor V, antithrombin III, immunoglobulin-G (IgG), immunoglobulin-M (IgM), immunoglobulin-A (IgA), a2-macroglobulin, angiogenin, Fg-D, elastase, keratinocyte growth factor (KGF), epidermal growth factor (EGF), fibroblast growth factor (FGF), tumour necrosis factor (TNF), interleukin-1 (IL-1), keratinocyte growth factor-2 (KGF-2), and combinations thereof.

In a preferred embodiment, the composition of the invention comprises one or more bone growth promoters selected from the group consisting of FGF, TGF-β, IGF-2, PDGF and BMP.

The bone growth promoter (E) may also comprise any combination of the exemplified compounds mentioned above.

The amount of the bone growth promoter (E) in the composition of the invention can be 0-10 % by weight of the total composition, such as 0-5 % by weight of the total composition, 0.1-5 % by weight of the total composition, or 0.2-4 % by weight of the total composition.

The compositions of the invention may be formulated such as to allow a local delivery of the antimicrobial agent (D), the bone growth promoter (E), drugs, proteins, enzymes, nutritional products, or other similar components. The composition may also comprise other additives, such as a filler, a reinforcing agent (*e.g.* metal fibres, synthetic fibres such as polyethylene fibres), ceramics, extracellular matrices (ECM), glycosaminoglycans, and polymer microspheres. Any of these additives may serve as a depot for local delivery of one or more of the antimicrobial agent (D), the bone growth promoter (E), drugs, proteins, enzymes, nutritional products, or similar components.

The composition of the invention can further comprise a solvent (F). The solvent (F) may be a solvent mixture. Suitable solvents include methanol, ethanol, and other suitable solvents known to those skilled in the art. Preferably, the solvent (F) comprises one or more alcohols. Preferably, the solvent comprises ethanol.

The amount of the solvent (F) in the composition of the invention can be 40-85 % by weight of the composition including solvent, such as 50-75 % by weight of the composition including solvent. In the case of three-dimensional printing applications, the amount of solvent (F) may be lower, such as from 0-60 % by weight of the composition including solvent, or from 0-40 % by weight of the composition including solvent.

In summary, the composition of the invention can suitably comprise:
- 10-90 % of the compound (A) by weight of the total composition, such as 10-80 % by weight of the total composition, 10-70% by weight of the total composition, 10-60 % by weight of the total composition, 10-50 % by weight of the total composition, 15-50 % by weight of the total composition, or 15-40 % by weight of the total composition;
- 3-50 % of the amine (B) by weight of the total composition, such as 3-40 % by weight of the total composition, 3-30 % by weight of the total composition, 3-20 % by weight of the total composition, or 4-15 % by weight of the total composition;
- 1-25 % of the compound (C) by weight of the total composition, such as 1-20 % by weight of the total composition, 1-15 % by weight of the total composition, 5-25 % by weight of the total composition, 5-20 % by weight of the total composition, 5-15 % by weight of the total composition or 2-10 % by weight of the total composition;
- 0-10 % of the antimicrobial agent (D) by weight of the total composition, such as 0-5 % by weight of the total composition
- 0-10 % of the bone growth promoter (E) by weight of the total composition, such as 0-5 % by weight of the total composition; and
- 40-85 % of the solvent (F) by weight of the composition including solvent, such as 50-75 % by weight of the composition including solvent.

The composition of the invention is preferably an osteoconductive composition and/or osteoconductive coating composition, and may be provided with osteoconductive properties upon addition of a suitable bone growth promoter (E). The term "osteoconductive" as used in this application is meant to refer to the ability of a substance or material to provide surfaces that facilitate new bone formation. The term "osteoinductive" as used in this application is meant to refer to the ability of substance or material to promote cellular functions that have the potential to stimulate new bone formation. Compositions according to the invention can be prepared by simply mixing the respective components. In case the compositions comprise an antimicrobial agent (D) and a solvent (F), it may be preferred to first dissolve the antimicrobial agent in the solvent prior to adding the other components of the composition into the solution of antimicrobial agent (D) and solvent (F).

The viscosity of the compositions of the invention can be adjusted to have a viscosity suitable for the chosen method of using the composition to make an article or coating, such as applying the composition as a coating.

In a further aspect, the invention is directed to a coating composition and to a coating that is prepared from a coating composition according to the invention.

The coating, after drying and curing, can have a thickness in the range of, for instance, 1-50 µm (micrometres), preferably in the range of 2-25 µm, such as in the range of 3-15 µm. In an embodiment, the coating has a thickness of from 1 µm to 2 mm.

The coating of the invention can be a coating that covers a substrate completely, but may also be a coating that incompletely covers the substrate. In the latter case, at least part of the surface of the substrate is not coated and is exposed. In an embodiment, a coating of the invention is selectively formed on a substrate. For example, the coating is formed in a specific shape or pattern, such as stripes on a substrate. The coating may be selectively formed to allow for a desired level of osteointegration. For example, in the case that repositioning of an implant is contemplated, the coating may be formed in stripes to result in slower or incomplete osteointegration. Further, should the composition contain the antimicrobial agent (D), the bone growth promoter (E), drugs, proteins, enzymes, nutritional products, or other similar components, one or more of these components may be distributed uniformly throughout the composition of selectively positioned in the composition.

The coating of the invention may be formed directly on a substrate, or can be a layer in a multi-layer coating of a substrate. In the latter case, the coating is preferably the outermost layer of the multi-layer coating. In an embodiment, a primer layer is applied on the substrate and the coating is formed on the primer layer. Washing of the substrate, for instance in a 5-10 % NaOH solution in water may alternatively be applied to improve adhesion of the coating to the substrate. In an embodiment, the substrate is plasma treated before forming the coating.

In yet a further aspect, the invention is directed to a medical device, such as an implant, comprising a substrate comprising a coating according to the invention.

The term "implant" as used in this application is meant to refer to a surgical implant suitable for use *in vivo.* More particularly, the implant can be an orthopaedic, trauma, or dental implant or prosthetic. Examples of suitable implants include total knee joints, total hip joints, ankle, elbow, wrist, and shoulder implants including those replacing or augmenting cartilage; long bone implants such as for fracture repair and external fixation of tibia, fibula, femur, radius and ulna; spinal implants including fixation and fusion devices; maxillofacial implants including cranial bone fixation devices, artificial bone replacements, orthopaedic cements and glues comprised of polymers, resins, metals, alloys, plastics and combinations thereof; nails, screws, plates, fixator devices, wires, sutures, and pins and the like that are used in such implants, and other orthopaedic implant structures as would be known to those of ordinary skill in the art. Alternatively or additionally, the implant can be a scaffold used to replace and generate bone.

Typically, implants are made of solid materials, either polymers, ceramics, metals, or combinations thereof.

The substrate of the implant that is coated with the coating of the invention may comprise any material from which implants are made of, including metals, ceramics and plastics. Examples of these materials include amorphous and/or (partially) crystalline carbon; complete carbon material; porous carbon; graphite; composite carbon materials; carbon fibres; ceramics such as calcium phosphates, zeolites, silicates, aluminium oxides, aluminosilicates, silicon carbide, and silicon nitride; clays, such as laponite; metal carbides; metal oxides; metal nitrides; metal carbonitrides; metal oxycarbides; metal oxynitridres and metal oxycarbonitrides of the transition metals (such as titanium, zirconium, hafnium, vanadium, niobium, tantalum, chromium, molybdenum, tungsten, manganese, rhenium, iron, cobalt, and nickel); metals and metal alloys of the noble metals gold, silver, ruthenium, rhodium, palladium, osmium, iridium, and platinum; metals and metal alloys of titanium, zirconium, hafnium, vanadium, niobium, tantalum, chromium, molybdenum, tungsten, manganese, rhenium, iron, cobalt, nickel and copper; steel, in particular stainless steel; shape memory alloys such as nitinol; nickel-titanium alloys; glass; stone; glass fibres; minerals; natural or synthetic bone substance; bone imitates based on alkaline earth metal carbonates such as calcium carbonate, magnesium carbonate, strontium carbonate and any desired combination of the above-mentioned materials.

Advantageously, the invention may allow direct bone apposition on plastic without the need of a metal substrate. Hence, in an embodiment the substrate is a plastic substrate. The substrate can hence include one or more polymers. Suitable examples of polymers include polyamides, polyphosphazenes, polypropylfumarates, polyethers, polyacetals, polycyanoacrylates, polyurethanes, polycarbonates, polyanhydrides, polyorthoesters, polyhydroxyacids, polyacrylates, ethylene vinyl acetate polymers, cellulose acetates and other cellulose derivates, polystyrenes, poly(vinyl chloride), poly(vinyl fluoride), poly(vinyl imidazole), poly(vinyl alcohol), polyesters such as poly(ethylene terephthalate) and polylactides, polyureas, polymethacrylates, polyolefins such as polyethylene and polypropylene, poly(ethylene oxide)s and chlorosulphonated polyolefins.

The substrate of the implant that is coated with the coating of the invention can be an ultrahigh molecular weight polyethylene (UHMWPE) substrate. More preferably, the implant is an UHMWPE implant. The substrate may also be ceramics, polyether ether ketone (PEEK), or polyether ketone ketone (PEKK).

In some embodiments, the substrate may comprise more than one type of material (*e.g.* may be comprised of a composite material).

In yet a further aspect, the invention is directed to a method for preparing an implant with a coating, said method comprising:
- coating at least part of the implant with a composition according to the invention,
- curing the composition, thereby forming a coating, and
- optionally, post-curing the coating, preferably by subjecting it to sterilisation.

The composition may be applied on a surface by any conventional coating method, such as spin coating, dip coating, spray coating, and the like. Generally, the composition will have a viscosity below 50 mPa·s if the composition is applied via spray coating. In some instances, the composition will have a viscosity below 30 mPa·s or even below 10 mPa·s. The viscosity of the composition may be adjusted by the content of the solvent.

The curing step is typically performed by thermal curing at a temperature in the range of 60-200 °C, preferably in the range of 70-150 °C, more preferably in the range of 80-120 °C. In an embodiment, the curing is performed at room temperature. The thermal curing step can last up to 7 hours, such as for 1-5 hours or 2-4 hours.

Optionally, a post-treatment like a post-curing step is performed. In an embodiment, the post-curing step is performed by subjecting the coated implant to sterilisation. Such a post-curing step may lead to a significant improvement of the mechanical stability of the coating. Suitable sterilisation methods may include autoclaving, ethylene oxide sterilisation, reactive nitrogen species (NO₂) sterilisation, electron beam irradiation, plasma gas sterilisation, or gamma irradiation. For example, the sterilisation can comprise autoclaving, involving a temperature of 100-150 °C, such as 110-140 °C and an appropriate pressure, such as an overpressure of 0.5-2 bar. The autoclaving may last for 5-40 minutes, such as 10-30 minutes. Suitably, the autoclaving involves the use of a saturated steam atmosphere. Preferably, the post-curing step is performed by autoclaving.

In yet a further aspect the invention is directed to a method for improving the bone apposition onto an implant, comprising coating said implant with a composition according to the invention.

In yet a further aspect, the invention is directed to a bone cement comprising the composition according to the invention. In an embodiment, a composition for a bone cement comprises the composition according to the invention.. In an embodiment, a composition for a bone cement comprises the composition according to the invention including a solvent (F). In an embodiment, the solvent (F) comprises ethanol.

In yet a further aspect, the invention is directed to an article formed from the composition according to the invention. In embodiments of the invention, the composition is cured into the shape of an article, preferably a medical device, or an implant. In an embodiment, the composition is placed in a mould, the composition is cured thereby forming an article, and the article is separated from the mould.

In yet a further aspect, the invention is directed to a liquid composition for three-dimensional printing, a method of forming a three-dimensional object, and a three-dimensional object formed via three-dimensional printing.

In an embodiment, a process of forming a three-dimensional object comprises the steps of selectively forming and curing a layer of the composition, and repeating the steps of selectively forming and curing a layer of the composition a plurality of times to obtain a three-dimensional object. A process capable of forming three-dimensional objects in this way is an ink-jet printing process.

In an embodiment, an ink-jet printer for three-dimensional printing comprises multiple jets, wherein at least one jet comprises a first liquid composition comprising the epoxy and not the amine, and at least one other jet comprises a second liquid composition comprising the amine and not the epoxy. Either jet may contain the component (C). A three-dimensional object may be formed by selectively jetting the first composition and the second composition according to a portion of the shape of a three-dimensional object. When the first composition and the second composition are mixed, they harden or cure and form a portion of the shape of a three-dimensional object.

In an embodiment, the mixing occurs in the time prior to the drops reaching the substrate or a previously formed portion of the three-dimensional object, for example by jetting the two drops toward each other so that they combine prior to reaching the substrate or a previously formed portion of the three-dimensional object. In an embodiment, a first drop (from the first liquid composition or second liquid composition) is first jetted onto the substrate or a previously formed portion of the three-dimensional object, and then a second drop (from the liquid composition that is not present in the first drop) is jetted onto the first drop that is already in position on the substrate or on a previously formed portion of the three-dimensional object.

In an embodiment, a process of forming a three-dimensional object comprises the steps of forming and selectively curing a layer of the composition, and repeating the steps of forming and selectively curing a layer of the composition a plurality of times to obtain a three-dimensional object. Optionally, the process of forming a three-dimensional object can further comprise a post-treatment in order to fully cure the composition and/or to further shape the three-dimensional object.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (*i.e.*, meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.*, "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. While certain optional features are described as embodiments of the invention, the description is meant to encompass and specifically disclose all combinations of these embodiments unless specifically indicated otherwise or physically impossible.

For the purpose of clarity and a concise description features are described herein as part of the same or separate embodiments, however, it will be appreciated that the scope of the invention may include embodiments having combinations of all or some of the features described.

The invention will now be further illustrated by means of the following examples, which are not intended to limit the scope in any manner.

### Examples

### Example 1 - Bone Integration

### Coating preparation

Compositions as described in Table 1 were prepared by first dissolving chlorhexidine diacetate (Chex DiAc) in ethanol (EtOH) followed by the addition of other components into the Chex DiAc EtOH solution. Compositions were prepared freshly immediately prior to application. Coatings were formed on medical grade titanium discs (diameter 2 cm, thickness 1 mm), which had previously been cleaned by submergence in ethanol and treatment with ultrasound for 5 minutes, followed by submergence in acetone and treatment with ultrasound for 5 minutes, followed by submergence in ethanol and treatment with ultrasound for 5 minutes once again. The composition was applied over the surface of a disc and subsequently the sample was spun at a rotation speed of 1000 rpm for 30 seconds. After drying on air (0.5-2 hours) the samples were placed in an oven at about 90 °C for 3 hours.

**Table 1. Example 1 Compositions**

| Composition | EtOH wt.% | Epoxy 1 ¹⁾ wt.% | Epoxy 2 ²⁾ wt.% | IPD ³⁾ wt.% | Dopamine wt.% | Chex DiAc wt.% |
|---|---|---|---|---|---|---|
| EpoCH1-0 | 66.1 | 22.0 | - | 7.6 | 4.3 | - |
| EpoCH1-5 | 65.0 | 21.6 | - | 7.5 | 4.2 | 1.7 |
| EpoCH1-10 | 63.8 | 21.4 | - | 7.3 | 4.1 | 3.4 |
| EpoCH2-0 | 62.5 | - | 26.2 | 7.3 | 4.0 | - |
| EpoCH2-5 | 61.6 | - | 25.7 | 7.1 | 4.0 | 1.7 |
| EpoCH2-10 | 60.4 | - | 25.5 | 7.0 | 3.9 | 3.3 |

| | | | | | | |
|---|---|---|---|---|---|---|
| ¹⁾ Epoxy 1: glycerol diglycidyl ether ²⁾ Epoxy 2: 1,4-cyclohexanedimethanol diglycidyl ether ³⁾ IPD: isophorone diamine | | | | | | |

Different types of coatings termed EpoCH1 and EpoCH2 (Table 1) have been prepared on titanium and aluminium substrates. The coatings showed fair mechanical properties (in rub tests) and good adhesion under dry and wet conditions.

### Bone integration of EpoCH1 coating on titanium rods evaluated in rabbits

The actual *in vivo* experiments were preluded by an *ex vivo* test series of 8 rabbits. The *ex vivo* evaluation of the surgical approach was performed in an animal cadaveric experiment. Two possible approaches were evaluated: medial arthrotomy and a transpatellar approach.

The medial arthrotomy proved to result in more tissue damage and an increased risk of damaging the medial collateral and cruciate ligaments. Furthermore, an arthrotomy also needs a larger incision. The transpatellar approach resulted in a simple and fast approach with little damage to the tendons and ligaments and above all the joint remained closed.

Titanium coated with the compositions EpoCH1-0, EpoCH1-5, and EpoCH1-10, as described in Table 1, were implanted in the tibia of rabbits. The composition was applied via dip coating. The coated titanium samples were then autoclaved.

After 6 weeks the animals were sacrificed and bone apposition on the implant surface was analysed by histology.

Histological analysis indicated that the titanium coated with EpoCH1-0 outperformed uncoated titanium with regard to bone apposition at the implant surface (71.5 % for uncoated titanium versus 98.8 % for the titanium coated with EpoCH1-0 after 6 weeks of implantation, respectively). The chlorhexidine-loaded coatings perform equally to the uncoated titanium surface. These data indicate the potential of the unloaded coating as osteoconductive coating with the potential capacity to serve as drug delivery system. In addition, these data suggest that the release of chlorhexidine does not hamper bone apposition as compared to uncoated titanium, but coatings loaded with the stated amounts of chlorhexidine perform less favourably compared to the same coating without chlorhexidine.

### Example 2 - Antimicrobial Agent Release

### Chlorhexidine Release

For the chlorhexidine release experiments, coatings were applied on 5 × 5 cm aluminium sheets by dip coating following the same procedure as described above. Then, 1 × 5 cm pieces were cut from these samples and incubated with 4 ml of phosphate buffered saline (pH = 7.0) at 37 °C under shaking at 100 rpm. At certain time intervals the medium was exchanged for fresh phosphate buffered saline, and the chlorhexidine concentration in the washing solution was determined by UV-VIS spectroscopy. The absorption values at λ = 255 nm were converted to mass concentrations of chlorhexidine via a calibration curve, and these values plotted against time in a cumulative way.

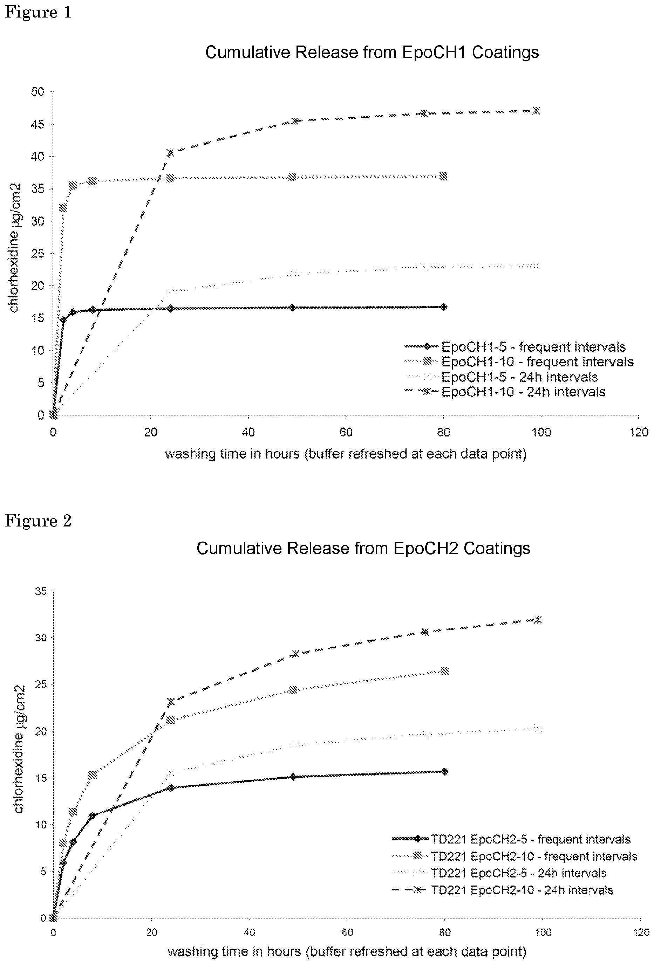

Figures 1 and 2 show the release of chlorhexidine from the EpoCH1 and EpoCH2 coatings. From the release curves, it can clearly be observed that the total amount of released chlorhexidine can be adjusted by changing the amount chlorhexidine in the composition. Further, the rate of release can be adjusted by the type of epoxy resin employed, with the more hydrophobic resin (EpoCH2) resulting in a slower release. The difference in release kinetics became most evident when the washing medium was changed frequently and more data points were collected (see the different curves for '24 h intervals' and 'frequent intervals' in figures 1 and 2).

### Silver Release

For the silver release experiments, coatings were applied on 5 × 5 cm stainless steel sheets by dip coating at approximately 19.2 °C and a speed of about 29 seconds per 50 cm. 45 cm² of the 50 cm² sheet was coated, leaving just a small uncoated section along one end. The coating formulation used in the silver release experiment is shown in Table 2. The coatings were cured at 90 °C for three hours. After curing, all samples except EpoAg-noAC were additionally subjected to autoclaving at 120 °C for 45 minutes in saturated steam. The EpoAg-noAC sample was put into an oven for 120 °C for 45 minutes in lieu of autoclaving.

After autoclaving, all samples were dipped in a solution of 0.066 g AgNO₃ in 106.1 g ethanol for 1 hour and then dried for 10 minutes at 50 °C.

The samples were incubated with 10 or 30 ml of phosphate buffered saline (pH = 7.0) at 37 °C under shaking at 100 rpm. At certain time intervals the medium was exchanged for fresh phosphate buffered saline, and the silver concentration in the washing solution was determined by ICP-AES. The ICP-AES results are converted to Ag concentrations, by making and measuring a calibration line, via a calibration curve, and these values (in mg Ag/L) plotted against time.

**Table 2. Coating compositions for silver release experiment**

| Composition | EtOH wt.% | Epoxy 1 ¹⁾ wt.% | IPD ³⁾ wt.% | Dopamine wt.% | Ag ⁴⁾ wt.% | AgNO₃ ⁵⁾ wt.% |
|---|---|---|---|---|---|---|
| EpoAg-1 | 76.08 | 15.06 | 5.08 | 3.45 | 0.32 | 0 |
| EpoAg-noAC | 76.09 | 15.09 | 5.09 | 3.41 | 0.32 | 0 |
| EpoAg-2 | 76.11 | 15.06 | 5.10 | 3.41 | 0.32 | 0 |
| EpoAgNO3 | 74.31 | 16.40 | 5.53 | 3.70 | 0 | 0.068 |

| | | | | | | |
|---|---|---|---|---|---|---|
| ¹⁾ Epoxy 1: glycerol diglycidyl ether ³⁾ IPD: isophorone diamine ⁴⁾ stabilized ionic silver nanoparticles ⁵⁾ AgNO₃ (Sigma) | | | | | | |

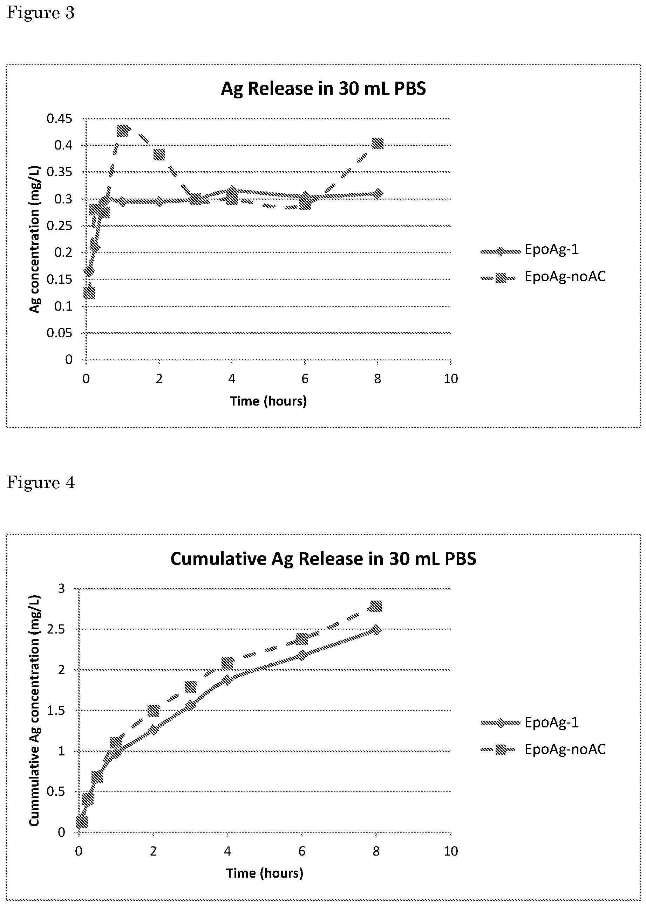

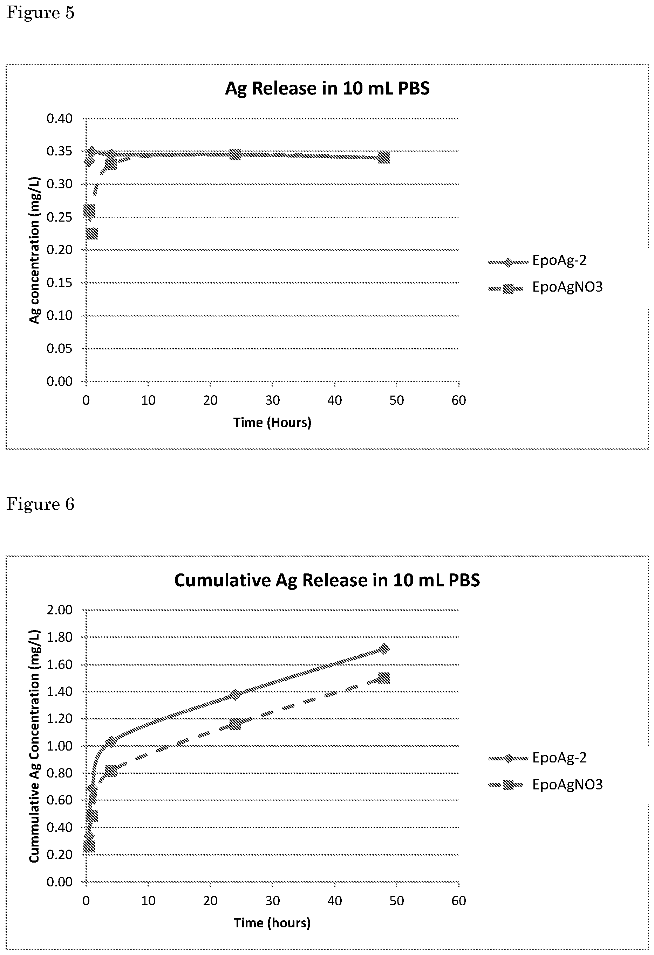

The results are presented in Figures 3-6. The samples show acceptable burst release of silver. However, autoclaving is found to remove some amount of silver, as shown by the lower silver release of the autoclaved sample. Given the osteointegrative properties demonstrated in Example 1, it is contemplated that the burst release of silver can provide a useful antimicrobial function and then, after release of substantially all of the silver, osteointegration proceeds.

### Example 3 - Mechanical Testing

### Coating preparation

The compositions shown in Table 3 were prepared by first dissolving stabilized ionic silver nanoparticles (Ag) in ethanol (EtOH) followed by the addition of other components in further ethanol. For DMTA tests, a cured film formed is by curing at 90 °C for three hours.

For the hardness and nanoindentation tests, the coating compositions are cured on 5 x 5 cm stainless steel plates having a thickness of 0.1 mm. Prior to coating composition application, the plates are cleaned by in an ultrasonic bath in six successive steps (10 minutes IPA, 10 minutes Milli-Q water and HNO₃, 10 minutes Demi water, 10 minutes Milli-Q water, 10 minutes 70/30 mixture of IPA/Milli-Q water, and 10 minutes drying at 80 °C). The coating composition is cured at 90 °C for three hours, followed by autoclaving in a saturated steam environment at 120 °C for 45 minutes.

**Table 3. Example 3 Compositions**

| Composition | EtOH wt.% | Epoxy 1 ¹⁾ wt.% | IPD ³⁾ wt.% | Dopamine wt.% | Ag ⁴⁾ wt.% |
|---|---|---|---|---|---|
| EpoAg0 | 74.62 | 16.29 | 5.49 | 3.68 | 0 |
| EpoAg1 | 74.54 | 16.27 | 5.49 | 3.68 | 0.03 |

| | | | | | |
|---|---|---|---|---|---|
| ¹⁾ Epoxy 1: glycerol diglycidyl ether ³⁾ IPD: isophorone diamine ⁴⁾ stabilized ionic silver nanoparticles | | | | | |

### E' and T_{g} by DMTA

The storage modulus (E') and glass transition temperature T_{g} are determined by DMTA as follows. The samples for the measurement are punched out of a cured film. The thickness is measured with a calibrated Heidenhain thickness meter. Typical sample size is a width of 2 mm, length between clamps of 25 mm, and a thickness varying between 1 and 2 mm. The dynamic mechanical measurements are performed in accordance with ASTM D5026 on equipment of the firm TA called RSA-G2 (Rheometrics Solids Analyser G2) at a frequency of 1 Hz and over a temperature area of -100 °C to 250 °C with a heating speed of 5 °C/min. The following deviations from ASTM D5026 are permitted: allowed temperature deviation ± 2 °C (in standard ± 1 °C), allowed force deviation ± 2 % (in norm standard ± 1 %), allowed frequency deviation ± 2 % (in standard ± 1 %), and heating speed 5 °C/min (in standard 1 to 2 °C/min). The T_{g} is determined as the temperature at which the loss modulus E" at a frequency of 1 Hz is at its maximum value.

The results presented are an average value across 3-5 samples. The results are shown in Table 4. E' is reported in MPa. T_{g} is reported in °C.

**Table 4. DMTA Results**

| Coating | E' (-40 °C) | E' (- 23 °C) | E' (37 °C) | E' (100 °C) | E' (150 °C) | E' (200 °C | T_{g} |
|---|---|---|---|---|---|---|---|
| EpoAg0 | 4019 | 24 | 9 | 4 | 5 | 10 | -6 |
| EpoAg1 | 3982 | 25 | 10 | 4 | 5 | 9 | -7 |
| EpoAg0 autoclaved | 4587 | 2522 | 584 | 5 | 6 | 10 | 33 |
| EpoAg1 autoclaved | 4353 | 474 | 50 | 5 | 6 | 9 | 16 |

Samples indicated as autoclaved were subjected to autoclave in a saturated steam environment at 120 °C for 45 minutes.

As can be seen, post-curing via autoclaving dramatically increases the mechanical properties of the system. The loading with silver as the anti-microbial agent has a somewhat negative effect on the E' of the coating and causes a reduction in the T_{g}.

### Nanoindentation and Hardness

The EpoAg1 (autoclaved) coating was subjected to a nanoindentation test. At an applied load of 10 mN, a Young's Modulus E of 62 GPa is measured at max. 10 % penetration of the coating thickness. Additional tests at 10 × the amount of silver by weight and 1/10^{th} the amount of silver by weight indicate that the Young's Modulus is not significantly affected by silver loading. The nanoindentation data shows a coating that adheres surprisingly well to the substrate, such that the coating may be suitable for use as a coating for an implant or other medical device. Hardness of the EpoAg1 (autoclaved) coating was also measured using a Vickers hardness test. The coating shows acceptable hardness and may be suitable for use as a coating for an implant or other medical device.

## Claims

1. A composition comprising:
(A) a compound comprising two or more epoxy groups;
(B) an aliphatic amine comprising one or more selected from a primary amine group and a secondary amine group; and
(C) one or more compounds selected from general formula (1), (2), (3), and (4) 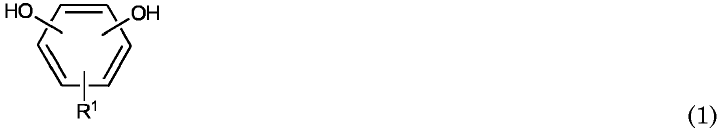 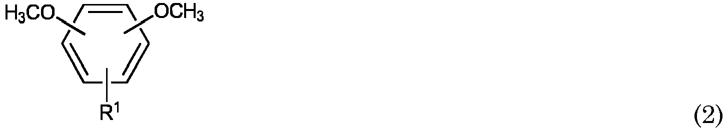 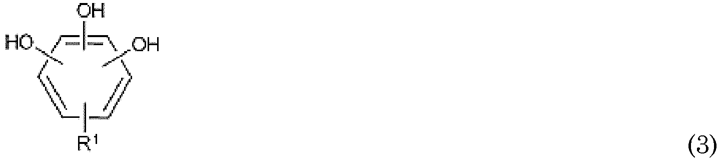 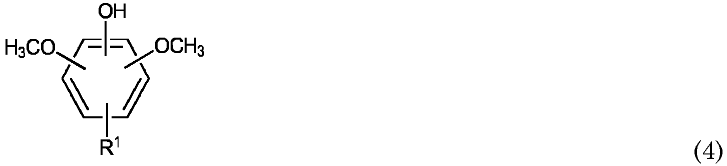
wherein R¹ is a group comprising a primary amine group, a secondary amine group, a carboxyl group, and/or a thiol group, and
wherein the sum of the number of epoxy groups in component (A) and the number of primary amine groups in component (B) is equal to three or greater.

2. The composition according to claim 1, wherein R¹ is a group comprising a primary amine group and/or a secondary amine group.

3. The composition according to claim 1 or 2, wherein component (C) is a compound selected from general formula (1) or (3).

4. The composition according to any one of claims 1-3, wherein component (C) is a compound of general formula (1).

5. The composition according to any one of claims 1-4, wherein the compound (A) is selected from the group consisting of 1,4-butanediol diglycidyl ether, 1,4-cyclohexanedimethanol diglycidyl ether, 4-vinylcyclohexene diepoxide, ethyleneglycol diglycidyl ether, hydrogenated diglycidyl ether of bisphenol A, glycerol diglycidyl ether, neopentylglycol diglycidyl ether, poly(ethyleneglycol) diglycidyl ether, and poly(propyleneglycol) diglycidyl ether, preferably said compound (A) is selected from the group consisting of 1,4-cyclohexanedimethanol diglycidyl ether, hydrogenated diglycidyl ether of bisphenol A, and glycerol diglycidyl ether.

6. The composition according to any one of claims 1-5, wherein said amine (B) is selected from the group consisting of 1,4-bis(3-aminopropyl) piperazine, 3,3'-diamino-*N*-methyl dipropylamine, 4,7,10-trioxa-1,13-tridecanediamine, ethylenediamine, isophorone diamine, and *N*-(2-aminoethyl)-1,3-propanediamine.

7. The composition according to any one of claims 1-6, wherein said amine is isophorone diamine.

8. The composition according to any one of claims 1-7, wherein the compound (C) is according to general formula (5) 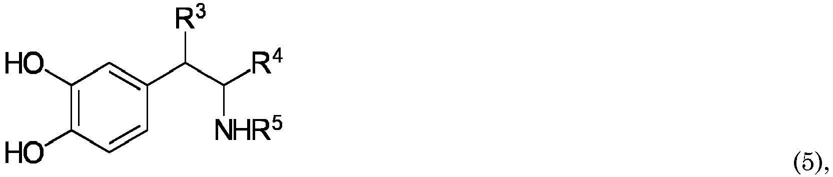 wherein
R³ is H, or OH,
R⁴ is H or COOH, and
R⁵ is H or CH₃.

9. The composition according to any one of claims 1-8, wherein said compound (C) is selected from the group consisting of *N*-methyldopamine, dopamine, epinephrine, norepinephrine, and L-dihydroxyphenylalanine.

10. The composition according to any one of claims 1-9, wherein said compound (C) is dopamine.

11. The composition according to any one of claims 1-10, wherein the equivalent ratio of all primary and secondary amine groups to all epoxy groups in the total composition is in the range of 0.95-1.10.

12. The composition according to any one of claims 1-11, wherein the equivalent ratio of all primary and secondary amine groups to all epoxy groups in the total composition is in the range of 1.00-1.08.

13. The composition according to any one of claims 1-12, further comprising
(D) an antimicrobial agent, preferably present at 0.01-10 % by weight of the total composition.

14. The composition according to claim 13, wherein said antimicrobial agent comprises silver nanoparticles, ionic silver, and/or a biguanide.

15. The composition according to claim 14, wherein said antimicrobial agent comprises silver nanoparticles and/or ionic silver.

16. The composition according to any one of claims 1-15, further comprising
(E) a bone growth promoter, preferably present at 0.01-10 % by weight of the total composition.

17. The composition according to claim 16, wherein said bone grown promoter is one or more selected from the group consisting of FGF, TGF-β, IGF-II, PDGF and BMP.

18. The composition according to any one of claims 1-17, further comprising a solvent (F), wherein said solvent comprises one or more alcohols.

19. The composition according to claim 18, wherein said solvent is present at 40-80 % by weight of the composition including solvent (F).

20. The composition according to any one of claims 1-19, wherein said composition comprises:
- 10-70 % of the compound (A) by weight of the total composition;
- 3-30 % of the amine (B) by weight of the total composition;
- 1-15 % of the compound (C) by weight of the total composition.

21. The composition according to any one of claims 1-20, wherein said composition is an osteoinductive composition.

22. The composition according to any one of claims 1-21, wherein said composition is an osteoconductive composition.

23. A coating prepared from the composition according to any one of claims 1-22.

24. The coating according to claim 23, wherein said coating has a thickness in the range of 1-50 µm, preferably in the range of 2-25 µm, such as in the range of 3-15 µm.

25. An implant comprising a substrate that is coated with a coating according to claim 23 or 24.

26. The implant according to claim 25, wherein said substrate is selected from the group consisting of metallic substrates, polymer-based substrates, and ceramic based substrates.

27. The implant according to claim 25 or 26, wherein said substrate is a metallic substrate selected from the group consisting of stainless steel, titanium, and aluminium.

28. A method for preparing an implant with a coating, said method comprising:
- coating at least part of the implant with a composition according to any one of claims 1-22,
- curing the composition, thereby forming a coating, and
- optionally, post-curing the coating.

29. The method according to claim 28, wherein said post-curing comprises subjecting the coating to sterilisation.

30. The method according to claim 29, wherein said post-curing comprises autoclaving.

31. A method for improving the bone apposition onto an implant, comprising coating said implant with a composition according to any one of claims 1-22.

32. A bone cement comprising a composition according to any one of claims 1-22.

33. An article formed from a composition according to any one of claims 1-22.

## Patentansprüche

1. Zusammensetzung, umfassend:
(A) eine Verbindung, die zwei oder mehr Epoxygruppen umfasst;
(B) ein aliphatisches Amin, das ein oder mehrere Amingruppen umfasst, die aus einer primären Amingruppe und einer sekundären Amingruppe ausgewählt sind; und
(C) eine oder mehrere Verbindungen, die aus den allgemeinen Formeln (1), (2), (3) und (4) 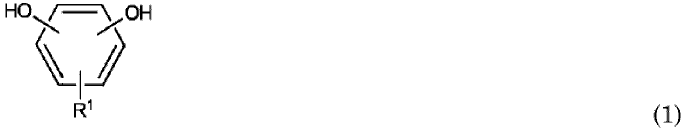 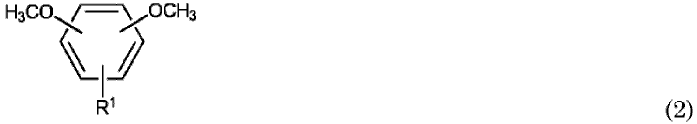 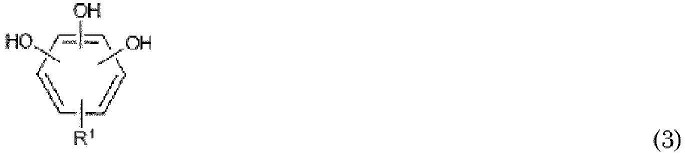 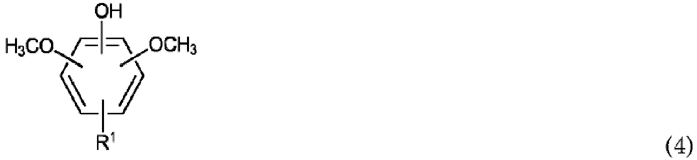 ausgewählt sind,
wobei es sich bei R¹ um eine Gruppe, die eine primäre Amingruppe, eine sekundäre Amingruppe, eine Carboxylgruppe und/oder eine Thiolgruppe umfasst, handelt und
wobei die Summe der Anzahl der Epoxygruppen in Komponente (A) und der Anzahl der primären Amingruppen in Komponente (B) gleich drei oder größer ist.

2. Zusammensetzung nach Anspruch 1, wobei es sich bei R¹ um eine Gruppe handelt, die eine primäre Amingruppe und/oder eine sekundäre Amingruppe umfasst.

3. Zusammensetzung nach Anspruch 1 oder 2, wobei es sich bei Komponente (C) um eine Verbindung handelt, die aus den allgemeinen Formeln (1) oder (3) ausgewählt ist.

4. Zusammensetzung nach einem der Ansprüche 1-3, wobei es sich bei Komponente (C) um eine Verbindung der allgemeinen Formel (1) handelt.

5. Zusammensetzung nach einem der Ansprüche 1-4, wobei die Verbindung (A) aus der Gruppe bestehend aus 1,4-Butandioldiglycidylether, 1,4-Cyclohexandimethanoldiglycidylether, 4-Vinylcyclohexendiepoxid, Ethylenglykoldiglycidylether, hydriertem Diglycidylether von Bisphenol A, Glycerindiglycidylether, Neopentylglykoldiglycidylether, Poly(ethylenglykol)diglycidylether und Poly(propylenglykol)diglycidylether ausgewählt ist, wobei die Verbindung (A) vorzugsweise aus der Gruppe bestehend aus 1,4-Cyclohexandimethanoldiglycidylether, hydriertem Diglycidylether von Bisphenol A und Glycerindiglycidylether ausgewählt ist.

6. Zusammensetzung nach einem der Ansprüche 1-5, wobei das Amin (B) aus der Gruppe bestehend aus 1,4-Bis(3-aminopropyl)piperazin, 3,3'-Diamino-N-methyldipropylamin, 4,7,10-Trioxa-1,13-tridecandiamin, Ethylendiamin, Isophorondiamin und N-(2-Aminoethyl)-1,3-propandiamin ausgewählt ist.

7. Zusammensetzung nach einem der Ansprüche 1-6, wobei es sich bei dem Amin um Isophorondiamin handelt.

8. Zusammensetzung nach einem der Ansprüche 1-7, wobei die Verbindung (C) der allgemeinen Formel (5) 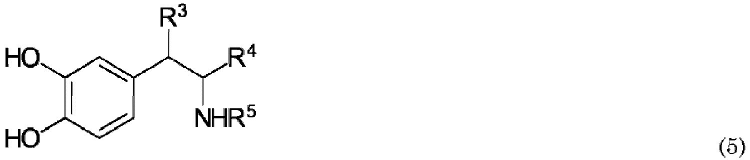 entspricht, wobei
R³ für H oder OH steht,
R⁴ für H oder COOH steht und
R⁵ für H oder CH₃ steht.

9. Zusammensetzung nach einem der Ansprüche 1-8, wobei die Verbindung (C) aus der Gruppe bestehend aus N-Methyldopamin, Dopamin, Epinephrin, Norepinephrin und L-Dihydroxyphenylalanin ausgewählt ist.

10. Zusammensetzung nach einem der Ansprüche 1-9, wobei es sich bei der Verbindung (C) um Dopamin handelt.

11. Zusammensetzung nach einem der Ansprüche 1-10, wobei das Äquivalenzverhältnis von allen primären und sekundären Amingruppen zu allen Epoxygruppen in der Gesamtzusammensetzung im Bereich von 0,95-1,10 liegt.

12. Zusammensetzung nach einem der Ansprüche 1-11, wobei das Äquivalenzverhältnis von allen primären und sekundären Amingruppen zu allen Epoxygruppen in der Gesamtzusammensetzung im Bereich von 0,95-1,10 liegt.

13. Zusammensetzung nach einem der Ansprüche 1-12, weiterhin umfassend
(D) ein antimikrobielles Mittel, vorzugsweise in einer Menge von 0,01-10 Gewichts-%, bezogen auf das Gewicht der Gesamtzusammensetzung.

14. Zusammensetzung nach Anspruch 13, wobei das antimikrobielle Mittel Silbernanopartikel, ionisches Silber und/oder ein Biguanid umfasst.

15. Zusammensetzung nach Anspruch 14, wobei das antimikrobielle Mittel Silbernanopartikel und/oder ionisches Silber umfasst.

16. Zusammensetzung nach einem der Ansprüche 1-15, weiterhin umfassend
(E) einen Knochenwachstumsförderer, vorzugsweise in einer Menge von 0,01-10 Gewichts-%, bezogen auf das Gewicht der Gesamtzusammensetzung.

17. Zusammensetzung nach Anspruch 16, wobei der Knochenwachstumsförderer aus der Gruppe bestehend aus FGF, TGF-β, IGF-II, PDGF und BMP ausgewählt ist.

18. Zusammensetzung nach einem der Ansprüche 1-17, weiterhin umfassend ein Lösungsmittel (F), wobei das Lösungsmittel einen oder mehrere Alkohole umfasst.

19. Zusammensetzung nach Anspruch 18, wobei das Lösungsmittel mit einem Anteil von 40-80 %, bezogen auf die Zusammensetzung einschließlich Lösungsmittel (F), vorliegt.

20. Zusammensetzung nach einem der Ansprüche 1-19, wobei die Zusammensetzung Folgendes umfasst:
- 10-70 %, bezogen auf das Gewicht der Gesamtzusammensetzung, Verbindung (A);
- 3-30 %, bezogen auf das Gewicht der Gesamtzusammensetzung, Amin (B);
- 1-15 %, bezogen auf das Gewicht der Gesamtzusammensetzung, Verbindung (C).

21. Zusammensetzung nach einem der Ansprüche 1-20, wobei es sich bei der Zusammensetzung um eine osteoinduktive Zusammensetzung handelt.

22. Zusammensetzung nach einem der Ansprüche 1-21, wobei es sich bei der Zusammensetzung um eine osteokonduktive Zusammensetzung handelt.

23. Beschichtung, hergestellt aus der Zusammensetzung nach einem der Ansprüche 1-22.

24. Beschichtung nach Anspruch 23, wobei die Beschichtung eine Dicke im Bereich von 1-50 µm, vorzugsweise im Bereich von 2-25 µm, aufweist, wie zum Beispiel im Bereich von 3-15 µm.

25. Implantat, umfassend ein Substrat, das mit einer Beschichtung nach Anspruch 23 oder 24 beschichtet ist.

26. Implantat nach Anspruch 25, wobei das Substrat aus der Gruppe bestehend aus metallischen Substraten, polymerbasierten Substraten und keramikbasierten Substraten ausgewählt ist.

27. Implantat nach Anspruch 25 oder 26, wobei es sich bei dem Substrat um ein metallisches Substrat handelt, das aus der Gruppe bestehend aus Edelstahl, Titan und Aluminium ausgewählt ist.

28. Verfahren zur Herstellung eines eine Beschichtung aufweisenden Implantats, wobei das Verfahren Folgendes umfasst:
- Beschichten von mindestens einem Teil des Implantats mit einer Zusammensetzung nach einem der Ansprüche 1-22,
- Aushärten der Zusammensetzung, wodurch eine Beschichtung gebildet wird, und
- gegebenenfalls Nachhärten der Beschichtung.

29. Verfahren nach Anspruch 28, wobei man beim Nachhärten die Beschichtung einer Sterilisation zu unterzieht.

30. Verfahren nach Anspruch 29, wobei das Nachhärten Autoklavieren umfasst.

31. Verfahren zur Verbesserung der Knochenapposition um ein Implantat, umfassend das Beschichten des Implantats mit einer Zusammensetzung nach einem der Ansprüche 1-22.

32. Knochenzement, der eine Zusammensetzung nach einem der Ansprüche 1-22 umfasst.

33. Gegenstand, der aus einer Zusammensetzung nach einem der Ansprüche 1-22 gebildet ist.

## Revendications

1. Composition comprenant :
(A) un composé comprenant deux groupes époxy ou plus ;
(B) une amine aliphatique comprenant un ou plusieurs groupes choisis parmi un groupe amine primaire et un groupe amine secondaire ; et
(C) un ou plusieurs composés choisis parmi la formule générale (1), (2), (3), et (4) 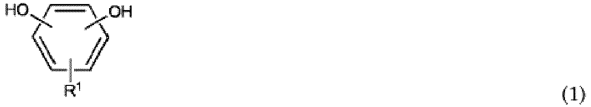 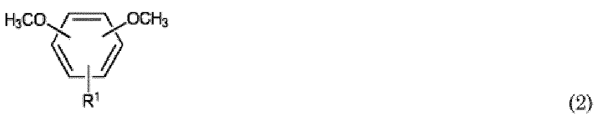 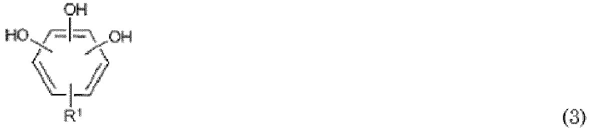 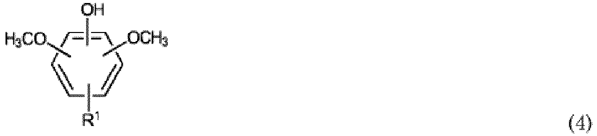
R¹ étant un groupe comprenant un groupe amine primaire, un groupe amine secondaire, un groupe carboxyle, et/ou un groupe thiol, et
la somme du nombre de groupes époxy dans le composant (A) et le nombre de groupes amine primaire dans le composant (B) étant égal à trois ou plus.

2. Composition selon la revendication 1, R¹ étant un groupe comprenant un groupe amine primaire et/ou un groupe amine secondaire.

3. Composition selon la revendication 1 ou 2, le composant (C) étant un composé choisi parmi la formule générale (1) et (3).

4. Composition selon l'une quelconque des revendications 1 à 3, le composant (C) étant un composé de formule générale (1).

5. Composition selon l'une quelconque des revendications 1 à 4, le composé (A) étant choisi dans le groupe constitué par le 1,4-butanediodiglycidyléther, le 1,4-cyclohexanediméthanoldiglycidyléther, le 4-vinylcyclohexènediépoxyde, l'éthylèneglycoldiglycidyléther, le diglycidyléther hydrogéné du bisphénol-A, le glycéroldiglycidyléther, le néopentylglycoldiglycidyléther, le poly(éthylèneglycol)diglycidyléther, et le poly(propylèneglycol)diglycidyléther, préférablement ledit composé (A) étant choisi dans le groupe constitué par le 1,4-cyclohexanediméthanoldiglycidyléther, le diglycidyléther hydrogéné du bisphénol-A, et le glycéroldiglycidyléther.

6. Composition selon l'une quelconque des revendications 1 à 5, ladite amine (B) étant choisie dans le groupe constitué par la 1,4-bis(3-aminopropyl)pipérazine, la 3,3'-diamino-N-méthyldipropylamine, la 4,7,10-trioxa-1,13-tridécanediamine, l'éthylènediamine, l'isophoronediamine, et la *N*-(2-aminoéthyl)-1,3-propanediamine.

7. Composition selon l'une quelconque des revendications 1 à 6, ladite amine étant l'isophoronediamine.

8. Composition selon l'une quelconque des revendications 1 à 7, le composé (C) correspondant à la formule générale (5) 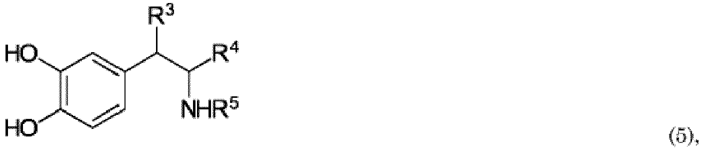
R³ étant H, ou OH,
R⁴ étant H ou COOH, et
R⁵ étant H ou CH₃.

9. Composition selon l'une quelconque des revendications 1 à 8, ledit composé (C) étant choisi dans le groupe constitué par la *N-*méthyldopamine, la dopamine, l'épinéphrine, la norépinéphrine, et la L-dihydroxyphénylalanine.

10. Composition selon l'une quelconque des revendications 1 à 9, ledit composé (C) étant la dopamine.

11. Composition selon l'une quelconque des revendications 1 à 10, le rapport équivalent entre tous les groupes amine primaire et secondaire et tous les groupes époxy dans la composition totale se situant dans la plage de 0,95 à 1,10.

12. Composition selon l'une quelconque des revendications 1 à 11, le rapport équivalent entre tous les groupes amine primaire et secondaire et tous les groupes époxy dans la composition totale se situant dans la plage de 1,00 à 1,08.

13. Composition selon l'une quelconque des revendications 1 à 12, comprenant en outre
(D) un agent antimicrobien, préférablement présent à raison de 0,01 à 10 % en poids de la composition totale.

14. Composition selon la revendication 13, ledit agent antimicrobien comprenant des nanoparticules d'argent, de l'argent ionique, et/ou un biguanide.

15. Composition selon la revendication 14, ledit agent antimicrobien comprenant des nanoparticules d'argent et/ou de l'argent ionique.

16. Composition selon l'une quelconque des revendications 1 à 15, comprenant en outre
(E) un promoteur de croissance osseuse, préférablement présent à raison de 0,01 à 10 % en poids de la composition totale.

17. Composition selon la revendication 16, ledit promoteur de croissance osseuse étant un ou plusieurs promoteurs choisis dans le groupe constitué par FGF, TGF-β, IGF-II, PDGF et BMP.

18. Composition selon l'une quelconque des revendications 1 à 17, comprenant en outre un solvant (F), ledit solvant comprenant un ou plusieurs alcools.

19. Composition selon la revendication 18, ledit solvant étant présent à raison de 40 à 80 % en poids de la composition comprenant le solvant (F).

20. Composition selon l'une quelconque des revendications 1 à 19, ladite composition comprenant :
- 10 à 70 % du composé (A) en poids de la composition totale ;
- 3 à 30 % de l'amine (B) en poids de la composition totale ;
- 1 à 15 % du composé (C) en poids de la composition totale.

21. Composition selon l'une quelconque des revendications 1 à 20, ladite composition étant une composition ostéoinductrice.

22. Composition selon l'une quelconque des revendications 1 à 21, ladite composition étant une composition ostéoconductrice.

23. Revêtement préparé à partir de la composition selon l'une quelconque des revendications 1 à 22.

24. Revêtement selon la revendication 23, ledit revêtement possédant une épaisseur dans la plage de 1 à 50 µm, préférablement dans la plage de 2 à 25 µm, tel que dans la plage de 3 à 15 µm.

25. Implant comprenant un substrat qui est revêtu par un revêtement selon la revendication 23 ou 24.

26. Implant selon la revendication 25, ledit substrat étant choisi dans le groupe constitué par des substrats métalliques, des substrats à base de polymère, et des substrats à base de céramique.

27. Implant selon la revendication 25 ou 26, ledit substrat étant un substrat métallique choisi dans le groupe constitué par l'acier inoxydable, le titane et l'aluminium.

28. Procédé pour la préparation d'un implant comportant un revêtement, ledit procédé comprenant :
- le revêtement d'au moins une partie de l'implant par une composition selon l'une quelconque des revendications 1 à 22,
- le durcissement de la composition, formant ainsi un revêtement, et
- éventuellement, le post-durcissement du revêtement.

29. Procédé selon la revendication 28, ledit post-durcissement comprenant la soumission du revêtement à une stérilisation.

30. Procédé selon la revendication 29, ledit post-durcissement comprenant un autoclavage.

31. Procédé pour l'amélioration de la pose d'un os sur un implant, comprenant le revêtement dudit implant par une composition selon l'une quelconque des revendications 1 à 22.

32. Ciment osseux comprenant une composition selon l'une quelconque des revendications 1 à 22.

33. Article formé à partir d'une composition selon l'une quelconque des revendications 1 à 22.
